# EUROPEAN PATENT APPLICATION

(11) **EP 2 008 688 A1**
(43) Date of publication of application: **31.12.2008**
(21) Application number: 08002720.4
(22) Date of filing: 14.02.2008
(51) Int. Cl.: A61N 2/00, A61N 5/067, A61N 2/06, A61N 5/06

(54) **Photo-magnetic radiation device**

(30) Priority: 26.06.2007 IL 18421807
(71) Applicant: Simenhaus, Zidkiyahu, 12155 Am (IL); Apostolou, Spiridon F., 15452 Athens (GR); Kaplunsky, Tamara, 11531 Kiryat Shemona (IL); Sheinman, Pablo, 47204 Ramat Hasharon (IL); Gertzman, Liora, 44444 Kfar Saba (IL)
(72) Inventor: Simenhaus, Zidkiyahu, 12155 Am (IL); Apostolou, Spiridon F., 15452 Athens (GR); Kaplunsky, Tamara, 11531 Kiryat Shemona (IL); Sheinman, Pablo, 47204 Ramat Hasharon (IL); Gertzman, Liora, 44444 Kfar Saba (IL)
(74) Representative: Neunert, Peter Andreas

(57) **Abstract**

There is provided a photo-magnetic radiation device (2), including a radiation emitting head (10) having a low level laser light source (42) for emitting light through a lightguide (20), a magnet (16) producing a magnetic field in the vicinity of the emitting head (10), a visible light emitter (40,41) and one infrared light source (39) located inside the emitting head (10), to emit visible and infrared lights together with the laser light and magnetic field. The device further includes a power source (12) and a controller (44) for selectively activating lights in the emitting head (10).

## Description

The present invention relates to photomagnetic therapeutic devices. More particularly the present invention is concerned with a laser light source combined with a magnetic field for therapeutic treatments and applications in tissues of human and animal bodies. The present invention could also be utilized for agricultural purposes, such as sterilizing seeds and stimulating plants growth.

### The Prior Art

Permanent magnets have been employed for a variety of therapeutic purposes. Beneficial effects have been observed utilizing magnets having a field strength from one Hertz to over 500 Hertz. Relative movement between the tissue and magnetic field has been shown to cause increase of electron flow (eddy current generation) through the tissue and is also associated with the activation of capillary blood flow and relaxation of muscles.

A further consideration in magnetic therapy devices is the selection of north pole versus south pole fields. Although magnetic fields are a continuous phenomena, there are subtle differences between north pole (or divergent) fields, south pole (or convergent) fields and mixed fields containing both divergent and convergent flux patterns.

The benefits of enhancing therapeutic effects by performance of dynamic fields may be achieved while preserving the orientation of polarity towards an individual by employing magnets in arrays that maintain such pole orientation. It is frequently desirable to maintain field orientation to deliver specific therapeutic applications with north pole or south pole fields directed at the individual. The magnetic fields will produce the intensified response of dynamic field while maintaining the polar orientation performance in the tissues of the individual.

Researches have demonstrated that magnetic fields have proven effective in a variety of therapeutic applications including relief of pain and inflammation from soft tissue injury, relief of headaches, treatment of internal organs, treatment of arthritis, increasing circulation and blood flow in the tissues subjected to the magnetic field and have also been claimed to dissolve calciferous plaques and deposits in the bodies of humans and animals. Additionally magnetic fields have been utilized to enhance and control the timing of plant growth.

U.S. Patent No. 5,389,981 to Riach Jr., discloses eyeglasses having a magnet attached thereto to project a north magnetic field into the surrounding area of the eyes to improve blood circulation. The device disclosed includes stationary magnets and intends to project a stationary magnetic field over the eyes.

U.S. Patent No. 4,177,796 to Franco-Vila, discloses a device for the treatment of arthritis which utilizes a pair of magnets, one magnet affixed to each side of an elastic band which is placed across the end of a housing containing an electromagnet. The paired magnets are caused to vibrate by application of a low frequency alternating magnetic field, causing the magnets and the elastic band to vibrate, so that, upon placing the end of the housing next to the injured tissue, vibration of the magnets acts to massage the tissue. The electromagnet subjects the tissue to be treated to a magnetic field, while the vibrating elastic band subjects the tissues to vibration.

U.S. Patent No. 4,682,584 to Pose, discloses a dental care instrument for treating emissions of foreign bodies within the mouth, the instrument having, at its operative end, a magnet which is placed within a patient's mouth. The end of the dental instrument is moved relative to the teeth. The user or individual providing treatment must move the dental care instrument with respect to the area to be treated to achieve vibrations of the magnet.

A review of the selected portion of the public knowledge discussed above reveals that magnetic therapy becomes an accepted and established modality for noninvasive and safe treatment that stimulates and claims accelerated healing processes.

The precise nature of the molecular events caused by using laser and LED's, i.e., by using low level light, near-infrared light; infrared and red light (LED's) irradiation, is still under investigating, however, laser therapy is currently used at medical centers for administering a variety of therapeutic treatments. Clinical evidence suggests that laser therapy accelerates healing processes including wound healing, pain relief, relaxation of muscles, reducing inflammation and increasing circulation and blood flow.

It is known in the art that biostimulation using low-energy laser, near-infrared light, red light and infrared light each promotes wound healing. Furthermore, many U.S. and other Patents disclose the use of light sources emitting various wavelengths (i.e., colors), for administering positive and beneficial treatments to living organisms mammals and accelerating plants growth, for example:

U.S. Patent No. 5,187,377, discloses LED arrays containing a substrate and two sets of LED's emitting different colors of light which can be used as light sources for facsimile or scanner devices. The first and second sets of LED's are connected in series so that a current alternatively flows through one set or the other, in an opposite direction. The two sets of LED's connected in series are further connected with each other.

U.S. Patent No. 5,500,009, discloses lasers and LED's in photo-therapy for the treatment of various ailments in humans. A method of treating herpes is disclosed using at least one LED emitting red light preferably an array of LED's which can be directed to concentrate the light. The voltage can be varied to vary the intensity of the light, and the lights can be pulsed.

U.S. Patent No. 5,358,503 discloses a photo-thermal therapeutic device using arrays of LED's for the simultaneous or selective treatment of areas of skin and adjacent subcutaneous structure in human subjects, utilizing photo energy and therapeutic heat. The LED array is held in a flexible or performed holder to provide contact with the skin. Heat, as well as light, are provided through the LED's. The intensity of the light and the heat can be varied. Resistors cause each LED to act as a heat sink during photo-therapy treatment. This patent cites U.S. Patent. Nos. 4,535,784 and 5,024,236, which disclose photo-therapy applied to human acupuncture points.

U.S. Patent No. 5,913,884, discusses a method for modulating wound healing in a mammal, which employs arrays of LED of laser irradiation applied after the administration of thee appropriate photo-sensitizer , which activate the light process.

U.S. Patent No. 5,634,711, discloses a hand-held portable light-emitting device suitable for photo-curing and photo-therapy applications. LED arrays are used, with means for varying the level of the light.

U.S. Patent No. 4,930,504, discloses devices and methods for bio-stimulation of tissues, comprising arrays of monochromatic radiation sources of multiple wavelengths. The radiation sources are arranged within the arrays so that radiation of at least two different wavelengths passes directly or indirectly through a single point within the treated tissue. Laser diodes or super-luminous diodes can be used as radiation sources. Controls are provided to turn the device on or off, vary pulse frequency and duration time of the treatment.

U.S. Patent No. 5,445,608, discusses various methods of photo-dynamic therapy, and discloses methods and apparatus for providing such therapy by employing an implantable probe to illuminate internal treatment sites which have been perfused with photo-reactive agents, including arrays of LED's or solid-state laser diodes.

U.S. Patent No. 5,660,461, discloses LED arrays assembled from pluralities of modular units which are snapped together. Reflector units are provided to direct the radiation. The modules can be electrically connected together in series or parallel. The arrays can be used to stimulate plant growth or for photo-dynamic therapy.

U.S. Patent No. 6,896,693 discloses a photo-therapy device comprising an array of LED's emitting visible and high intensity infrared light in five predetermined different wave lengths 470 nm, 565 nm, 590 nm, 630 nm and 880 nm. One device allows both cold and heat treatment. The red-wave frequency (visible light, i.e., blue frequency) - cold treatment, and the IR wave frequency causes a thermal effect. The device is intend to lie against the skin or a surface near the skin/surface, and/or a distance ranging up to several feet, from the skin/or surface.

A review of the selected portion of the prior art discussed above shows that photo-therapy become an accepted and established modality for non-invasive and safe treatment of bums, cuts, inflammations, the stimulation of sedation of acupressure meridian points in human and animals, bone fracture repair, acceleration of blood flow, aiding relaxation of the nervous system and also stimulation of plant growth.

It should be noted, according to the accumulative public knowledge achieved via treatment of patients at hospitals and clinical studies, that laser therapy (i.e., Low Level Laser therapy or LLLT) generally requires the injured tissue to be exposed directly to the laser light for predetermined intervals of time. Exposure to laser light not only lessens the pain associated with certain disorders, but actually speeds the healing of the treated tissues. The wavelength of the laser light, the intensity of the laser light and the exposure time are important factors when selecting a specific protocol for a specific disorder.

The wavelength of laser light affects its ability to penetrate through the tissues and molecules of interest. For example, red light is attenuated by most tissues, and thus the penetration depth is less than 1 cm into such tissues, whereas near-infrared (NIR) is less attenuated by most tissues, and thus can penetrate more than 1 cm into such tissues. These penetration limits are a drawback, and therefore, the typically target tissue for laser therapy are subcutaneous tissues.

The wavelength of the laser light also affects its ability to promote biological pathway for healing injured tissues. For example the quantum energy of NIR photons is small, and thus NIR photons have a relatively low potential to electronically exciting biomolecules. On the other hand, the quantum energy of red wavelength photons is sufficient to achieve electronic excitation of biomolecules, potentially promoting direct photochemical and photobiological effects in target tissues.

It is well known in the art that magnetic radiation is utilized for therapy purposes. Magnetic fields are also known to increase the penetration ability into cell membranes in human and animal tissues subjected to the magnet field, as well as enhancing and/or controlling plant growth.

It is noted that 85% of the human body is formed by the fluid media. Subjected to the impact of the permanent magnetic field of the present invention, the dipoles of the biological molecules of the fluid media of the body, change from a disordered state into an oriented state, which align themselves with the power lines of the external magnetic field. This newly formed phenomenon could also be described as a sort of magnetic field whose dipoles have been arranged in vertical lines, forming "pathways", wherein the molecules function as a non-linear optical medium.

As a result of the north - south dipoles rebalanced state of the electromagnetic energy-field surrounding the targeted tissues (or organs), the potential of laser lights to penetrate via the "pathways" into molecules in deep tissues and organs, is significantly upgraded in comparison to such cases where there are no continuous bio-magnetic impacts and the dipoles of the biological molecules are not arranged.

Moreover, whereas the programmed direction of the radiated laser lights coincides with the direction of the oriented biological molecules' dipoles, which coincides with the magnetic power lines, the penetration of the coincided directed laser-wave-lights via the formed "pathways", i.e., along the arranged biological vertical lines into deep targeted tissues and organs, is significantly higher than the penetration ability provided by prior art devices.

Studies which have been carried out demonstrate that in such occurrence of the bio-magnetic phenomenon, the ability of the low level laser light of the present invention to penetrate into the depth, has been increased up to 7 cm. The super short laser pulse of the soliton wave having a duration ranging between (40 - 110) x 10-⁹sec. with a front pulse rise of (5 - 15) x 10⁻⁹sec., penetrates directly and effectively into some soft tissues of even up to 15 cm.

In addition, as a result of the occurrence of the above described phenomenon, since the biological tissues have optical properties including transparency, the penetration ability of the near-infrared light, infrared light and red lights, at the wavelength spectrum rates above-indicated, into deep tissues and organs, is also significantly increased, e.g., the IR penetrates up to 10 cm and even up to 13 cm.

Such penetration performances of the laser lights into deep tissues and organs as has been demonstrated by the present invention allows, at the same time, a coincided coordinated reduction of the duration of the laser wave lights' pulses and a reduction of the time duration of the treatment pattern, without decreasing the therapeutic effectiveness. The reduction of the dose radiation delivered to the patient, as well as the reduction of the time treatment, minimizes potential risks and/or side effects.

A further significant advantage of the present invention over prior art devices is the ability to provide a simultaneous coordinated irradiation supplied by five energy sources, i.e., the low level laser light, near-infrared light, infrared light, red light and a magnetic impact, all simultaneously irradiated from one single device.

Hence, the present invention, when used, provides therapeutic effects derived of the quality impacts of each one of the five acting factors: the magnetic field, low level laser light, near-infrared, infrared lights and red lights. Furthermore, an added advantage of the present invention over prior art devices results from the simultaneously combined activity of the five acting factors, above described. Thus, the device according to the present invention generates a synergy effect that enhances the therapeutic effectiveness and potentates influence upon the biological structure of an object, i.e., a patient, animal or a plant.

In accordance with the present invention there is provided a photo-magnetic radiation device, comprising a radiation emitting head including a low level laser light source for emitting light through a lightguide, a magnet producing a magnetic field in the vicinity of said emitting head and at least one visible light emitter and one infrared light source located inside said emitting head to emit visible and infrared lights together with said laser light and magnetic field, a power source, and a controller for selectively activating lights in the emitting head.

### Brief Description of the Drawings

The invention will now be described in connection with certain preferred embodiments with reference to the following illustrative figures so that it may be more fully understood.

With specific reference now to the figures in detail, it is stressed that the particulars shown are by way of example and for purpose of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Fig. 1 is a perspective top view of the magnetic/optical radiation device, according to the present invention;
Fig. 2 is a rear view of the device of Fig. 1;
Fig. 3 is an exploded perspective top view of the device of Fig. 1;
Fig. 4 is a top view of an embodiment illustrating the relative dispositions of the active component of the device;
Figs. 5A to 5E are schematic views of several embodiments of lightguides utilizable with the device, according to the present invention, and
Fig. 6 is a perspective view of another embodiment of the device, according to the present invention.

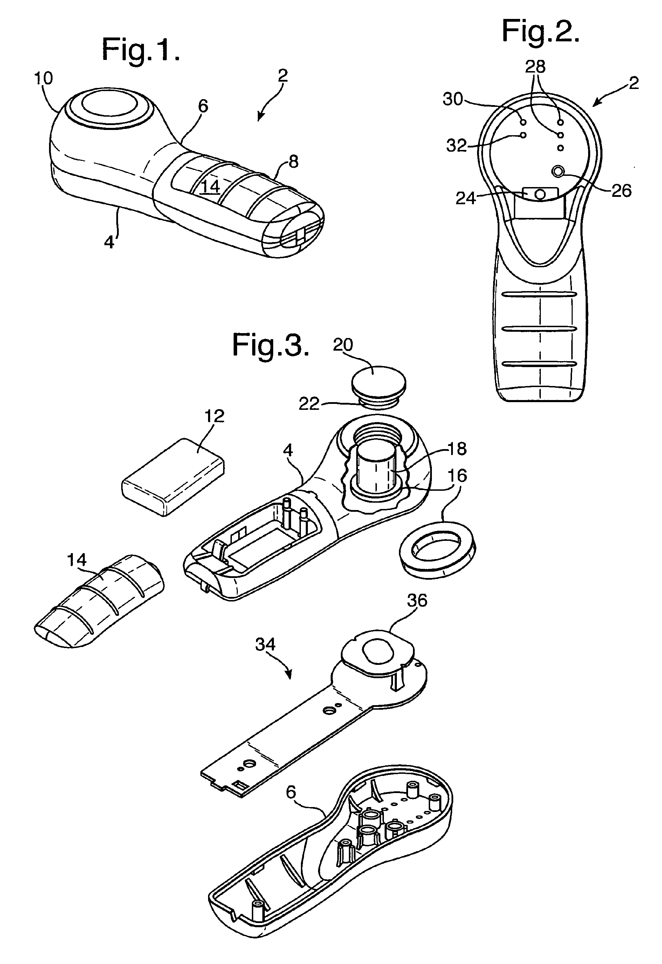

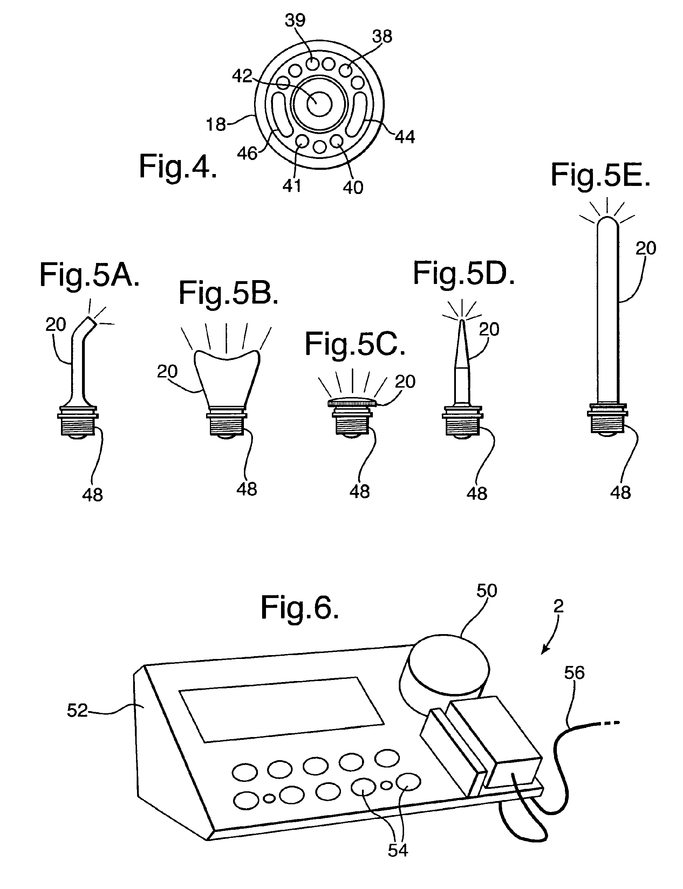

A preferred embodiment of the magnetic and optical radiation-emitting device 2, according to the present invention, is illustrated in Figs. 1 to 3. The device 2 consists of a two-part housing 4, 6 forming a handle portion 8 and a head portion 10. The handle portion is configured to house a power source 12, e.g., a rechargeable battery, accessible through a removable cover 14. The head portion 10 is designed to enclose an annular magnet 16 surrounding a light passing conduit 18. A lightguide 20, having a threaded base 22 closes the top opening of the conduit 18. At the rear side of the device 2 (Fig. 2), there are distributed operating elements including an ON/OFF switch 24, a mode selector 26, as well as, advantageously, light indicators 28 displaying the fact that the drive is in operating mode, the state of the power source (indicator 30) the radiated frequency (inductor 32), and the like. The housing part 6 also serves as a base to which is attached a panel 34 (Fig. 3) supporting on a plate 36, radiation-emitting diodes, including IR LEDs 39, Red LEDs 40, and optionally, NIR and Blue LEDS, 38, 41, all encircling a laser 42 (Fig. 4).

As can be seen in Fig. 4, the laser 42 is disposed in the center of the plate 36, the LEDs are distributed around the laser 42, allowing light prevailing inside the conduit 18 to exit through the lightguide 20. The plate 36 also carries a controller 44, a processor 46, including operation programs, and a connector to load programs from a computer, as required.

Referring to Figs. 5A to 5G, there are illustrated various embodiments of lightguides 20, which can be utilized with the device 2. Each of the lightguides 20 is configured differently and has a base with a connector 48, e.g., a screw thread, a bionet connector, or the like, for connecting same to the top of the head 6, above and contiguous to the conduit 18.

Turning now to Fig. 6, there is shown a modification of the device 2, in which the device is divided into two parts: a hand-held manipulatable part 50, which includes a radiation emitter and a stationary part 52, accommodating operation and control components 54, as well as, optionally, a cable connector 56 to the mains, instead of the built-in power source 8 shown in Fig. 1.

The nature and functions of the various active components of which the device 2 is composed, are as follows:

The magnet 16 is preferably a magnet producing a permanent magnetic field of a strength that varies from 5 to 40 milliTesla (mT), which orients the axes of bio-molecular magnetic dipoles of a living tissue to increase internal energy of molecules. It also allows keeping the ionized molecules in dissociated state. This increases efficiency of other curative factors of quantum therapy at molecular and cellular levels. The magnetic field is applied in concert with other trapped radiation, as will be described hereinafter. The laser 42 comprises a laser diode which, for example, irradiates low level laser light at a wavelength of 750 - 1200 nm.

An alternate embodiment of the present invention comprises a laser array that generates a super short laser pulse. The time duration of the super short laser pulse ranges between (40 - 110) x 10⁻⁹sec., with a front pulse rise of (5 - 15) x 10⁻⁹sec. The laser wave pattern, i.e., the pulse of a short duration and a steep leading front, is of the soliton monochrome type wave.

The non-coherent light includes several lights from which the operator can choose one or more lights to be radiated together with the laser radiation and the permanent magnetic field. The NIR light 38 (Fig. 4) emits pulses at a wavelength of 850 nm to 1050 nm. For the purpose of rendering a higher therapeutic effect on the deeply located soft tissues or organs while decreasing at the same time the dose radiated to the patient, the pulse power of the NIR light is coordinated to the range of 25 W - 100 W. Thus, the programmed on/off time ratio of the pulses could be considerably increased up and at the same time pulse duration could be sharply reduced. In the NIR spectrum rate the biological tissues are optically transparent.

The IR LEDs 39 provide non-coherent infrared light(s) in short pulses, at wavelengths that vary from 800 nm up to 1300 nm. Its duration is many times (hundreds up to thousands) more than that of the laser pulses, and the rear front of the infra-red pulse coincides with the phase of the laser probe.

The Red LEDs 40, which generate red light, i.e., visible light pulses, at wavelengths of 450 - 700 nm, emit average radiation output ranging between 5-200 mW.

Blue LEDs 41 generate blue light pulses at wavelengths around 472 nm. In some uses, the blue light replaces the NIR or the Red lights for predetermined treatments. Consequently, the application field of the present invention is expanded and the specific desired therapeutic impact is increased.

While the embodiments illustrated utilize a manipulatable device having a graspable hand and an integral radiation emitting head, the invention could also be embodied by a stationary radiation-emitting device, which is merely operated to radiate on a living tissue which is brought to traverse the radiation path.

It will be evident to those skilled in the art that the invention is not limited to the details of the forgoing illustrative embodiments and examples and that the present invention may be embodied in other specific forms without departing from essential attributes thereof, and it is therefore desired that the present embodiments and examples be considered in all respects as illustrative and not restrictive, reference being made to the appended claims, rather to the foregoing description, and all changes will come within the meaning and range of equivalency of the claims are therefore intended to be embraced therein.

## Claims

1. A photo-magnetic radiation device, comprising:
a radiation emitting head including a low level laser light source for emitting light through a lightguide;
a magnet producing a magnetic field in the vicinity of said emitting head and at least one visible light emitter and one infrared light source located inside said emitting head to emit visible and infrared lights together with said laser light and magnetic field;
a power source, and
a controller for selectively activating lights in the emitting head.

2. The device as claimed in claim 1, wherein said magnet is an annular magnet surrounding said light guide.

3. The device as claimed in claim 1, wherein said magnet is a permanent magnet.

4. The device as claimed in claim 1, further comprising blue and red and near infrared (NIR) light sources.

5. The device as claimed in claim 1, wherein said light sources are LEDs.

6. The device as claimed in claim 1, wherein said magnet emits a magnetic field of 5 to 40 mT.

7. The device as claimed in claim 1, wherein said light sources are arranged in an array.

8. The device as claimed in claim 1, wherein said laser emits pulses of wavelengths between 750 nm to 1200 nm.

9. The device as claimed in claim 8, wherein said laser emits pulses having a time duration of between (40 x 110) x 10⁻⁹ sec.

10. The device as claimed in claim 9, wherein said pulse has a rising pulse front of (5 - 15) x 10⁻⁹ sec.

11. The device as claimed in claim 4, wherein the pulse of said NIR range between 25 W to 100 W.

12. The device as claimed in claim 4, wherein average radiation output of said red light is between 5 and 200 mW.

13. The device as claimed in claim 1, wherein said laser radiates at a frequency range of between 1 Hz to 5000 Hz.

14. The device as claimed in claim 1, wherein the penetration depth of said laser light into a living tissue is up to 7cm.

15. The device as claimed in claim 1, wherein the penetration depth of a soliton laser light into a living tissue is up to 15 cm.

16. The device as claimed in claim 1, wherein the penetration depth of said infrared light into a living tissue is up to 13 cm.
